# FASCICULE DE BREVET EUROPEEN

(11) **EP 0 769 929 B1**
(45) Date de publication et mention de la délivrance du brevet: **06.05.1999**
(21) Numéro de dépôt: 95924383.3
(22) Date de dépôt: 29.06.1995
(51) Int. Cl.: A61F 2/06, A61M 31/00

(54) **DISPOSITIF THERAPEUTIQUE DESTINE AU TRAITEMENT CYTOREDUCTEUR SELECTIF DE L'OBSTRUCTION D'UNE LUMIERE OU VOIE NATURELLE D'UN CORPS HUMAIN OU ANIMAL**
THERAPEUTISCHE VORRICHTUNG ZUR ZYTOREDUKTIONSBEHANDLUNG EINER OBSTRUKTION EINES MENSCHLICHEN ODER TIERISCHEN KÖRPERGANGES
THERAPEUTIC DEVICE FOR THE SELECTIVE CYTOREDUCTION TREATMENT OF AN OBSTRUCTION IN A NATURAL LUMEN OR PASSAGE OF THE HUMAN OR ANIMAL BODY

(30) Priorité: 13.07.1994 FR 9408933
(43) Date de publication de la demande: 02.05.1997
(73) Titulaire: Devonec, Marian, F-01700 Miribel (FR)
(72) Inventeur: Devonec, Marian, F-01700 Miribel (FR)
(74) Mandataire: Guerre, Dominique
(86) Numéro de dépôt international: FR9500869
(87) Numéro de publication internationale: WO9602210

(56) Documents cités:
- EP-A- 0 164 241
- EP-A- 0 274 846
- WO-A-80/01460
- WO-A-89/03232
- US-A- 5 269 802

## Description

La présente invention concerne le traitement d'une lumière ou voie naturelle disposée dans une partie pleine, notamment dans un organe solide d'un corps humain ou animal, lumière par laquelle s'effectue un transit ou une circulation d'un fluide, notamment d'un fluide corporel, liquide ou gazeux, cette lumière ou voie naturelle étant obstruée sous l'effet d'une prolifération cellulaire locale.

Les voies urinaires, et notamment l'urètre, constituent par exemple des lumières naturelles au sens de la présente invention.

Par "prolifération cellulaire locale", on entend tout processus biologique, par exemple de type tumoral bénin ou malin, conduisant localement à un excédent tissulaire, organisé ou non, et provoquant une obstruction, ou obstruant la lumière ou voie naturelle considérée, à l'endroit où se développe ladite prolifération. L'hypertrophie bénigne de la prostate ou adénome prostatique constitue un exemple d'une telle prolifération cellulaire obstructive.

La présente invention sera introduite, définie et décrite, à titre d'exemple non limitatif, par référence au traitement des obstacles prostatiques aigus ou chroniques chez l'homme.

Aujourd'hui, on sait traiter l'obstacle prostatique de manière mécanique, c'est-à-dire sans action curative vis-à-vis de la cause de l'obstruction, et à cette fin, différentes prothèses endo-urétrales ont été décrites ou sont disponibles sur le marché. On se réfèrera à titre d'exemple à la prothèse décrite dans le document WO-A-94/18907.

On se réfèrera également au document WO-A-80/01460 qui décrit un dispositif endo-prothétique, destiné à un traitement mécanique, local et sélectif, de l'obstruction d'une lumière ou voie naturelle de circulation d'un fluide, disposée dans une partie pleine, à savoir dans un organe solide d'un corps humain ou animal, par exemple de l'obstruction d'un canal biliaire obstrué sous l'effet d'une prolifération cellulaire locale.

Ce dispositif comprend :
- un élément tubulaire, de forme cylindrique, non biodégradable, par exemple en matière plastique, agencé pour être placé dans la lumière naturelle, suffisamment souple pour se conformer à ladite lumière, mais suffisamment rigide pour maintenir un passage artificiel dans cette lumière ;
- un ou plusieurs manchons, supportes par cet élément tubulaire, positionnés selon la longueur et autour de ce dernier pour venir par expansion en regard et au contact direct de l'obstruction, et assurer l'autostatisme de l'élément tubulaire, une fois la lumière naturelle intubée par ce dernier.

De telles prothèses, implantées de manière permanente ou temporaire, n'apportent qu'un traitement palliatif des obstacles prostatiques.

Pour le patient, ces prothèses peuvent être mal tolérées, du fait de leur action purement mécanique, s'agissant de corps étrangers laissés en place en permanence, ou de façon temporaire mais répétée. Il peut exister dans certains cas un risque d'infection et de migration non négligeable. Ces prothèses, lorsqu'elles sont permanentes, présentent un risque d'obstruction, soit par incrustation (dépôt de cristaux contenus dans l'urine), soit par hyperplasie à l'intérieur de la prothèse, en réaction au corps étranger qu'elle représente ; cette hyperplasie peut aller jusqu'à obstruer certaines prothèses permanentes.

La présente invention a pour objet de remédier à ces inconvénients.

Plus précisément, l'invention a pour objet un traitement de l'obstruction d'une lumière naturelle, limité dans le temps, de type curatif, à action locale et sélective.

Conformément à la présente invention, on propose un nouveau dispositif de traitement thérapeutique, implantable dans la lumière ou voie naturelle à traiter, comprenant :
- de manière connue en soi, un élément tubulaire, non biodégradable, agencé pour être placé et retenu, de manière sensiblement auto-statique, dans la lumière naturelle traitée ; cet élément, préférentiellement de forme cylindrique, est à la fois suffisamment souple pour se conformer à la lumière naturelle contre sa paroi, et suffisamment rigide pour maintenir un passage artificiel, et donc une circulation dans la lumière naturelle ;
- de manière nouvelle, un manchon médicamenteux, supporté par l'élément tubulaire, positionné selon la longueur et autour de ce dernier pour venir en regard et au contact direct de l'obstruction, une fois la lumière naturelle intubée avec ledit élément tubulaire ; ce manchon comprend ou incorpore un agent thérapeutique cytoréducteur, notamment cytotoxique spécifiquement vis-à-vis des cellules de ladite prolifération cellulaire locale, essentiellement par simple contact tissulaire, superficiel et solide, avec lesdites cellules ; ce manchon est agencé par ailleurs pour délivrer cet agent thérapeutique, au moins dans sa partie externe.

Par "supporté par", on entend selon la présente invention que le manchon médicamenteux est présent sur l'élément tubulaire, soit de manière apparente et/ou distincte de ce dernier, soit de manière invisible, en étant compris ou incorporé dans ledit élément tubulaire, dans sa matière ou ses éléments constitutifs, sur une longueur et/ou à une position, prédéterminées dudit élément tubulaire.

Par "thérapeutique", on entend selon la présente invention, tout traitement de type médicamenteux, et notamment chimique, isolé ou complémentaire à un autre traitement, permettant localement de réduire de manière sélective l'obstruction de la lumière ou voie naturelle considérée, qu'il s'agisse des cellules tapissant la paroi de ladite lumière, ou qu'il s'agisse des cellules situées en arrière et en profondeur par rapport à ces dernières. Ce traitement de type médicamenteux est facilité ou complété selon la présente invention par un traitement de type mécanique, visant à maintenir pendant le traitement, puis rétablir l'écoulement dans la lumière naturelle, perturbé ou empêché en raison de l'obstruction de cette même lumière.

Grâce à l'invention, une fois le dispositif thérapeutique disposé dans la lumière naturelle obstruée, le manchon médicamenteux délivre sélectivement l'agent cytoréducteur, à la partie obstruée de la paroi de la lumière naturelle, et ensuite à la prolifération cellulaire sous-jacente. En conséquence de cette délivrance, on assiste à une érosion progressive de la partie obstruée de la paroi de la lumière naturelle traitée, puis du tissu sous-jacent responsable de la compression ou obstruction de cette même lumière, selon un front ayant la forme d'une enveloppe cylindrique, progressant radialement vers l'extérieur, de manière sensiblement concentrique au manchon médicamenteux. On aboutit ainsi à la formation d'un passage au travers de la lumière traitée, ayant une dimension transversale, au moins supérieure à la dimension transversale normale de la même lumière. Dès que cesse le contact entre la partie superficielle externe du manchon médicamenteux, et les tissus ou cellules environnantes, l'action cytoréductrice cesse, étant entendu que cette dernière peut cesser également, par simple extraction du dispositif thérapeutique selon l'invention de la lumière naturelle qu'il intube.

Au total, on aboutit à un pharmaco-modelage de la lumière naturelle traitée, laissant subsister un passage en quelque sorte "moulé" sur l'élément tubulaire du dispositif thérapeutique selon l'invention.

Par conséquent, par un double effet, à la fois médicamenteux et mécanique du dispositif selon l'invention, on peut ainsi construire un néo-passage dans la partie obstruée de toute lumière naturelle, ayant sensiblement le même axe et la même forme que l'élément tubulaire ayant servi à le former. Une fois ce néo-passage créé, le dispositif selon l'invention est retiré, et le néo-canal ainsi obtenu s'épithélialise, à partir des parties sus et sous-jacentes de la lumière naturelle non traitée par le manchon médicamenteux.

Préférentiellement, et bien qu'il s'agisse d'un mode d'exécution de l'invention parmi d'autres, d'une part, l'élément tubulaire comprend une âme interne, par exemple cylindrique, en un matériau biocompatible, notamment relativement lisse et mou, par exemple en un caoutchouc silicone, et d'autre part, le manchon médicamenteux, distinct de l'élément tubulaire et recouvrant ce dernier, est disposé à l'extérieur de l'âme, et comprend un substrat ou support biologiquement compatible, incorporant l'agent thérapeutique cytoréducteur.

Un dispositif thérapeutique selon l'invention présente encore les avantages essentiels suivants.

L'agent thérapeutique cytoréducteur est délivré in situ et spécifiquement à la prolifération cellulaire, générant ou ayant généré l'obstruction dans la lumière naturelle. Une telle administration locale d'un principe actif cytoréducteur diminue considérablement les effets secondaires ou morbidité, par rapport à une administration du même principe actif, par voie générale, par exemple par voie orale, s'agissant du traitement d'une hypertrophie bénigne.

L'effet cytoréducteur est strictement limité à la partie obstruée, à l'exclusion des zones environnantes de la lumière naturelle, qui demeurent à l'écart de l'action de l'agent thérapeutique cytoréducteur, puisque l'effet corrosif dudit agent sur les tissus environnants ne se produit que par contact mécanique ou solide, sans intermédiaire liquide.

Les produits de dégradation tissulaire ou cellulaire sont éliminés le long de la lumière naturelle, dont l'obstacle est supprimé. En général, ces produits ou déchets peuvent être dilués et évacués avec le fluide corporel circulant dans la lumière naturelle traitée.

Un dispositif thérapeutique selon l'invention peut être mis en place dans la lumière traitée de manière simple et atraumatique.

S'agissant du traitement thérapeutique de la partie prostatique de l'urètre de l'homme, un dispositif thérapeutique endo-urétral selon l'invention comprend deux éléments tubulaires, destinés à être disposés dans l'urètre, respectivement de part et d'autre du sphincter, et attachés l'un à l'autre par un moyen de liaison, souple et déformable, destiné à être pris dans l'orifice du sphincter. L'élément tubulaire supérieur supporte le manchon médicamenteux dans la partie prostatique de l'urètre, et l'élément tubulaire inférieur ne comporte pas de manchon médicamenteux. En particulier, le manchon médicamenteux est positionné par rapport à l'élément tubulaire supérieur, d'une extrémité dite inférieure, située au-dessus de l'extrémité basse (par rapport à la position implantée) de l'élément tubulaire supérieur, par exemple à environ 10 mm de cette extrémité basse, a une extrémité dite supérieure, située en retrait par rapport à l'extrémité haute de l'élément tubulaire supérieur, par exemple à une distance comprise entre 10 et 15 mm de cette extrémité haute.

Grâce à ces dispositions complémentaires pour un dispositif endo-urétral, l'effet cytoréducteur est limité à la zone d'obstacle qui s'étend du veru montanum au col de la vessie, et notamment au segment sus-montanal de l'urètre prostatique.

La partie de l'élément tubulaire supérieur, susceptible de dépasser dans la vessie une fois l'implant mis en place, n'est pas dangereuse, dans la mesure ou cette extrémité, pouvant venir en contact avec la paroi de la vessie, est dépourvue d'agent cytoréducteur sur une certaine longueur.

Un dispositif thérapeutique endo-urétral selon l'invention se distingue donc fondamentalement du dispositif endo-urétral décrit dans le document WO-A-89 03232, par le fait que :
- il permet de traiter une lumière ou canal anatomique, en l'occurrence l'urètre, obstrué, présent dans un organe solide ou plein, en l'occurrence la prostate, et non pas de manière générale une cavité anatomique, en l'occurrence la vessie, remplie avec un liquide servant à la diffusion de l'agent thérapeutique ;
- le support de l'agent thérapeutique ou principe actif utilisé est disposé le long et autour de l'élément tubulaire, et non pas à l'extrémité libre de ce dernier, laquelle reste selon la présente invention, dépourvue dudit agent thérapeutique ;
- l'action de l'agent thérapeutique s'exerce par simple contact tissulaire, superficiel et solide, avec les cellules à détruire, et non pas par diffusion dans un liquide, qui à son tour baigne la paroi à traiter ; par exemple, mais de manière non exclusive, tout agent thérapeutique non hydrosoluble peut être retenu selon la présente invention.

Et un dispositif thérapeutique selon l'invention se distingue fondamentalement du dispositif décrit par le document EP-A-0 164 241, par le fait que l'élément tubulaire est agencé pour être auto-statique, et non pas libre dans une cavité anatomique, comme la panse d'un ruminant, et pour assurer à l'intérieur la circulation d'un fluide, et non pas contenir un bloc biodégradable dans lequel est incorporé l'agent thérapeutique.

Toutes ces différences structurelles et fonctionnelles par rapport à l'art antérieur identifié précédemment, justifient notamment la novation fondamentale apportée par la présente invention, et résidant en particulier dans la notion de pharmaco-modelage d'un canal ou lumière naturelle d'un organe plein ou solide, décrite et définie ci-dessus.

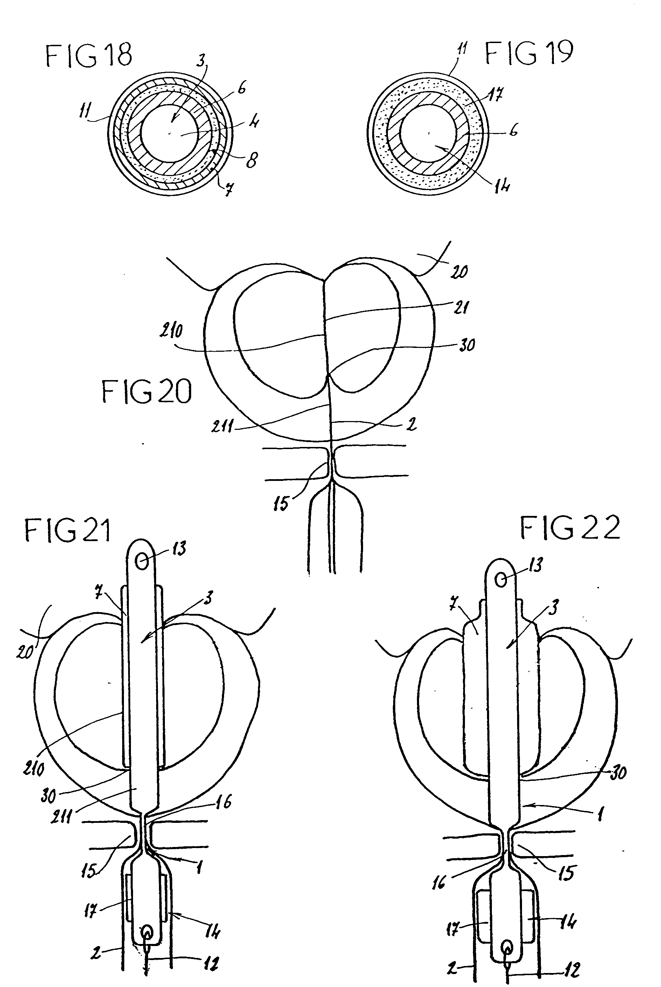

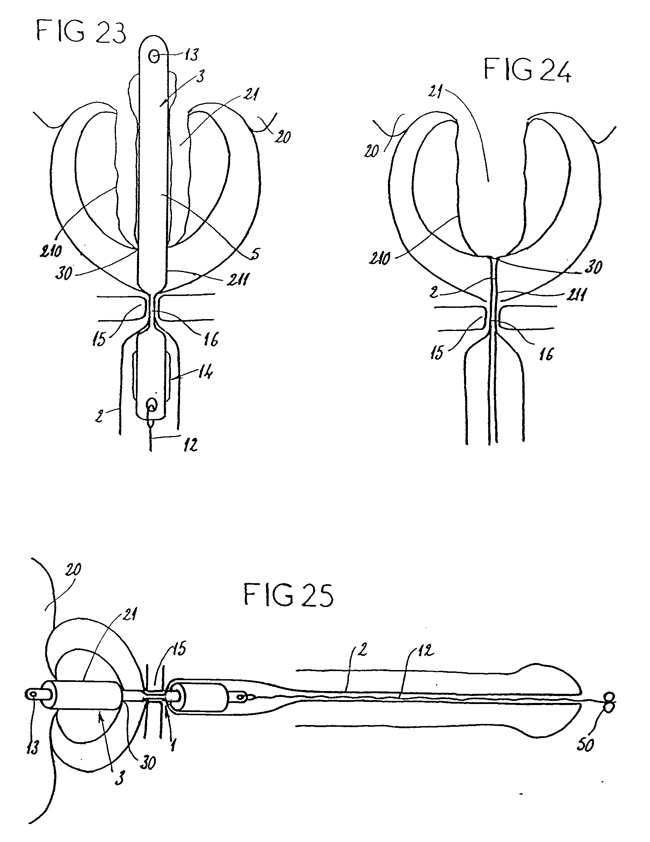

La présente invention est maintenant décrite par référence au dessin annexé, dans lequel :
- la figure 1 représente une coupe anatomique sagittale des voies urinaires du corps humain masculin ; un dispositif thérapeutique selon la présente invention est représenté sur cette coupe, en place dans l'urètre ;
- les figures 2 et 3 représentent un dispositif thérapeutique selon la présente invention, vu de face, avec son extrémité amont en haut et son extrémité aval en bas, respectivement dans sa configuration avant implantation, c'est-à-dire prêt à l'emploi, et dans sa configuration après implantation et activation, c'est-à-dire dans l'urètre, mais ce dernier n'étant pas représenté sur la figure 3 ;
- la figure 4 représente une vue en coupe transversale du dispositif représenté à la figure 2, selon le plan de coupe IV-IV, pris respectivement dans les éléments tubulaires supérieur et inférieur, avant implantation ;
- la figure 5 représente une vue en coupe transversale du dispositif représenté à la figure 2, selon le plan de coupe V-V, pris respectivement dans les éléments tubulaires inférieur et supérieur ;
- la figure 6 représente une vue en coupe transversale du dispositif représenté à la figure 3, après implantation et activation, selon le plan de coupe VI-VI, pris respectivement dans les éléments tubulaires inférieur et supérieur du dispositif ;
- la figure 7 représente une vue en coupe axiale du dispositif représenté à la figure 3, selon le plan de coupe VII-VII, pris respectivement dans les éléments tubulaires inférieur et supérieur du dispositif ;
- la figure 8 représente, à la manière de la figure 2, un autre mode d'exécution d'un dispositif selon l'invention ;
- la figure 9 représente une vue en coupe axiale du dispositif représenté à la figure 8, selon le plan de coupe IX-IX ;
- les figures 10 et 11 représentent en coupe axiale, un autre mode d'exécution d'un dispositif thérapeutique selon la présente invention, respectivement avant implantation et après implantation et activation dans l'urètre, ce dernier n'étant pas représenté ;
- les figures 12 et 13 représentent deux coupes transversales d'une variante d'exécution d'un dispositif représenté à la figure 10, avant implantation, respectivement selon les plans de coupe XII-XII et XIII-XIII identifiés à la figure 10 ;
- les figures 14 et 15 représentent des vues en coupe transversale du dispositif représenté aux figures 12 et 13, respectivement selon les plans de coupe XIV-XIV et XV-XV identifiés à la figure 11, après implantation et activation ;
- pour une variante d'exécution du dispositif thérapeutique représenté aux figures 12 à 15, les figures 16 et 17 représentent des vues de coupe axiale, respectivement selon les plans de coupe XII-XII et XIII-XIII, identifiés à la figure 10, avant implantation ;
- par rapport respectivement aux plans de coupe XII-XII et XIII-XIII, identifiés par référence à la figure 10, les figures 18 et 19 représentent des vues axiales d'un autre mode d'exécution d'un dispositif thérapeutique selon l'invention, avant implantation ;
- de manière schématique et en vue frontale, les figures 20 à 24 représentent l'urètre avant implantation conformément à la figure 20, le mode d'action d'un dispositif thérapeutique selon l'invention, mis en place conformément aux figures 21 à 23, et l'urètre après extraction du même dispositif, conformément à la figure 24 ;
- la figure 25 représente, en position implantée in vivo, un dispositif thérapeutique selon l'invention, représenté avec son fil d'extraction ou d'arrimage ;

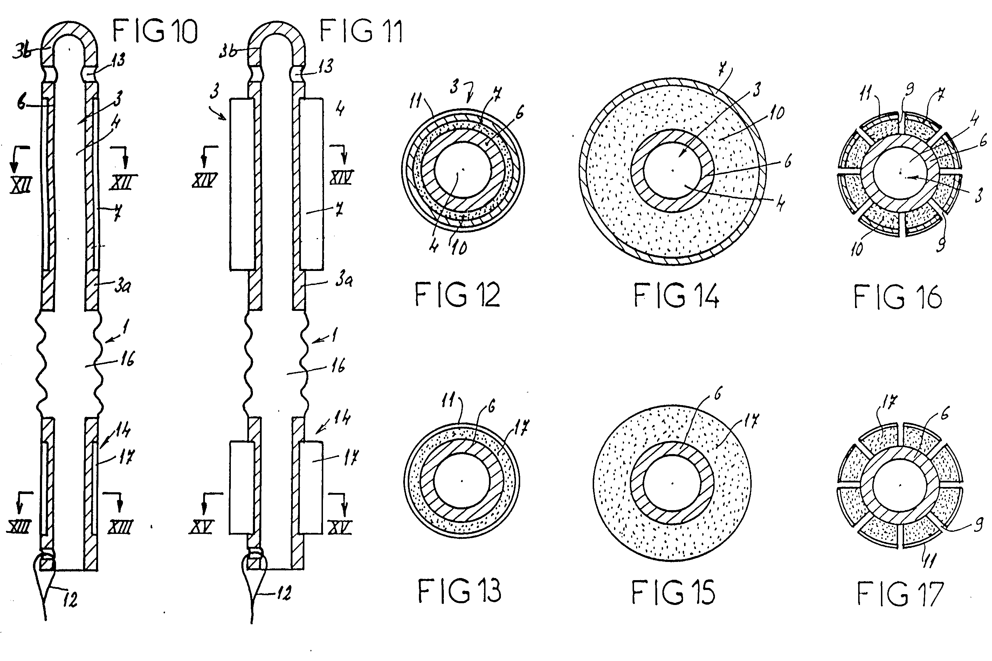

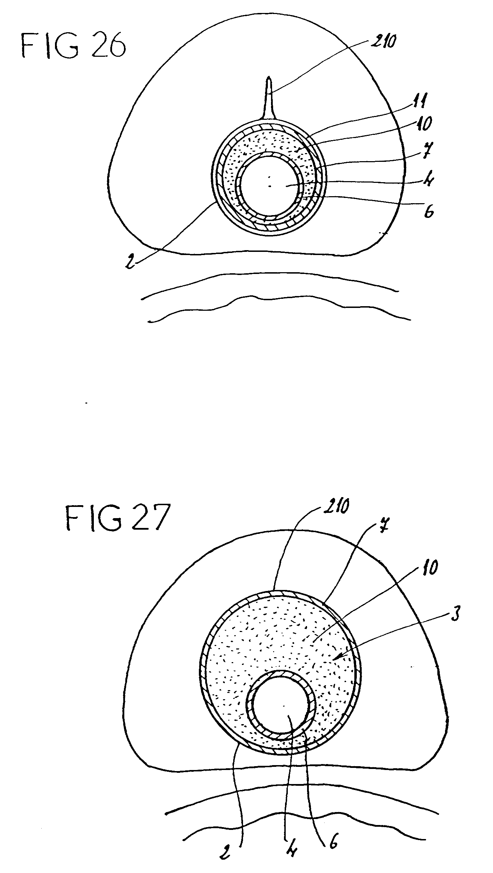

- les figures 26 et 27 représentent in vivo, et en coupe transversale, respectivement selon les plans de coupe XII-XII de la figure 10, et XIV-XIV de la figure 11, un autre mode d'exécution d'un dispositif thérapeutique selon l'invention, avant activation du dispositif selon la figure 26, et après activation du dispositif selon la figure 27.

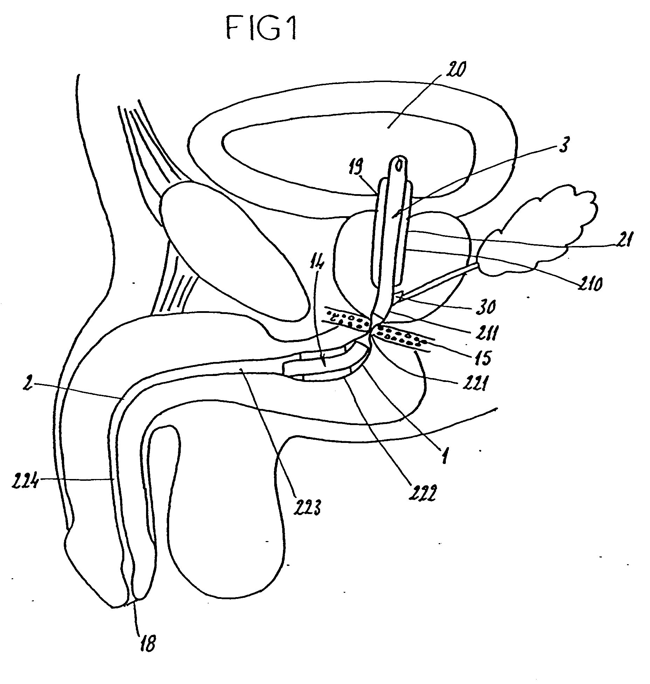

Conformément à la figure 1, l'urètre 2 s'étend de bas en haut, à partir du méat urinaire 18 jusqu'au col 19 de la vessie 20. Au-dessus du sphincter strié 15, l'urètre comprend une partie prostatique 21, composée d'un segment prostatique sus-montanal 210, et d'un segment prostatique sous-montanal 211, de part et d'autre du veru montanum 30 de la prostate. Au-dessous du sphincter 15, l'urètre comprend vers le méat 18, le segment membraneux 221, le segment bulbaire 222, le segment périnéal 223, et enfin le segment pénien 224.

En conséquence de tout adénome développé au niveau de la partie prostatique, cette dernière est susceptible de s'obstruer, en sorte qu'un dispositif thérapeutique conforme à la description ci-après doit être mis en place dans la partie 21.

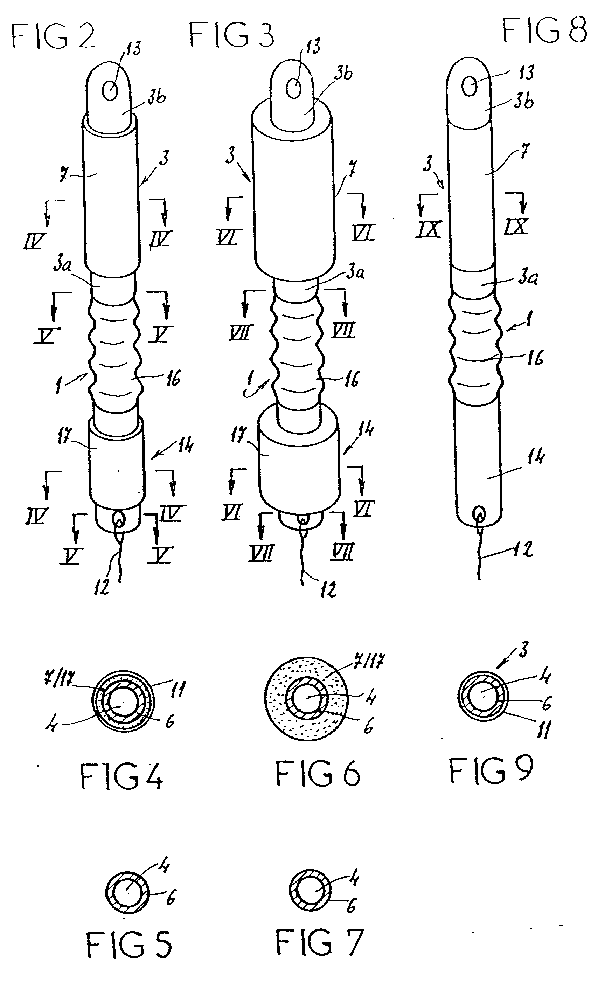

Conformément aux figures 2 et 3, ce dispositif thérapeutique 1 endo-urétral comprend de manière générale deux éléments tubulaires 3 et 14, destinés à être disposés dans l'urètre 2, respectivement de part et d'autre du sphincter 15, et attachés l'un à l'autre par un moyen 16 de liaison, souple et déformable, consistant en un manchon souple et élastique, destiné à être pris dans l'orifice du sphincter 15. Le manchon souple 16 est en continuité d'écoulement avec les éléments tubulaires 3 et 14.

L'élément tubulaire supérieur 3, destiné à se placer et être retenu dans la partie 21 prostatique de l'urètre, supporte et est recouvert par un manchon médicamenteux 7, positionné selon la longueur et autour dudit élément tubulaire 3. En conséquence, lorsque cet élément tubulaire 3 est disposé dans la partie 21 prostatique de l'urètre, le manchon médicamenteux 7 vient en regard et au contact direct de l'obstruction prostatique. Ce manchon médicamenteux 7 comprend un agent thérapeutique cytoréducteur, spécifiquement vis-à-vis des cellules de la prolifération cellulaire prostatique, essentiellement par simple contact tissulaire, superficiel et solide, avec lesdites cellules. Le manchon 7 est agencé par ailleurs, notamment en ce qui concerne son support, pour délivrer cet agent thérapeutique, dans sa partie superficielle externe ; cette délivrance en surface de l'agent thérapeutique peut être obtenue selon tous modes appropriés, bien connus de l'homme du métier, par exemple de manière quasi-instantanée, ou préférentiellement de manière lente, retardée ou progressive, en ajustant la nature et/ou la composition du substrat du manchon médicamenteux, servant en quelque sorte d'excipient à l'agent thérapeutique proprement dit.

L'autre élément tubulaire inférieur 14 ne comporte aucun manchon thérapeutique, et par conséquent ne concourt a aucun traitement thérapeutique de l'urètre, au-dessous du sphincter.

Les éléments tubulaires 3 et 14, par exemple de forme cylindrique, sont suffisamment souples pour se conformer à l'urètre, dans la position implantée, mais suffisamment rigides pour maintenir un passage artificiel 4 dans l'urètre, assurant la circulation de l'urine.

Le manchon thérapeutique 7 est positionné par rapport à l'élément tubulaire supérieur 3, selon sa longueur, en sorte qu'une extrémité dite inférieure est située au-dessus de l'extrémité basse 3a de l'élément tubulaire supérieur 3, par exemple à environ 10 mm de cette extrémité basse, et une extrémité dite supérieure est située en retrait et au-dessous de l'extrémité haute 3b de l'élément tubulaire supérieur 3, par exemple à une distance comprise entre 10 et 15 mm de l'extrémité haute précitée.

Comme montré aux figures 2 et 3, l'extrémité haute 3b de l'élément tubulaire supérieur 3 est borgne, et perforée 13 pour assurer le passage de l'urine à partir de la vessie 20.

Les éléments tubulaires 3 et 14 comprennent chacun une âme 6, sous la forme d'un tube en un matériau biocompatible, mais non biodégradable, notamment relativement lisse et mou, par exemple en un caoutchouc silicone.

Le manchon médicamenteux 7, revêt l'âme interne 6 de l'élément tubulaire supérieur 4, et est disposé à l'extérieur de cette dernière. Ce manchon 7 comprend un substrat biologiquement compatible, incorporant l'agent thérapeutique cytoréducteur. Ce substrat est expansible et éventuellement compressible radialement, en sorte que, à l'état sec et avant implantation du dispositif, il adopte une conformation ramassée et compacte, non expansée, et qu'après implantation et activation, à l'état mouillé ou humide, il adopte une conformation expansée ; ces deux conformations respectivement non expansées et expansées sont représentées respectivement aux figures 2 et 4, et 3 et 6. Le substrat du manchon 7 est par exemple une matière hydrophile, expansible sous l'effet des fluides biologiques présents ou circulant dans l'urètre. A titre d'exemple d'un tel substrat expansible et hydrophile, on retiendra différentes matières cellulosiques, déjà utilisées dans le domaine médical.

De manière similaire, mais sans agent thérapeutique cytoréducteur, l'élément tubulaire inférieur 14 supporte et est entouré par un manchon 17 extérieur, revêtissant l'âme interne 6 dudit élément 14, expansible et éventuellement compressible radialement, composé par exemple de la même matière hydrophile et expansible, et éventuellement biodégradable, que celle utilisée dans la composition du manchon médicamenteux 7.

Le manchon médicamenteux 7 est recouvert au départ, c'est-à-dire avant l'implantation du dispositif, par une enveloppe 11 superficielle protectrice, délitable et/ou biodégradable par contact tissulaire in situ avec la partie obstruée du canal de l'urètre. Plus précisément, cette enveloppe 11 est susceptible de retenir, à l'état non expansé, le manchon médicamenteux 7, avant l'implantation du dispositif, puis de se déliter, en laissant toute liberté à l'expansion du manchon 7 ; c'est ce que représentent respectivement les figures 2 et 4, et 3 et 6. Exactement de la même manière, une enveloppe 11 superficielle protectrice, délitable, peut être utilisée pour contenir, puis libérer le manchon extérieur 17 expansible entourant l'élément tubulaire inférieur 14.

Comme montre aux figures 2 et 3, mais aussi à la figure 25, un fil d'extraction ou d'arrimage 12 est arrimé à l'extrémité aval du dispositif, et plus précisément à l'extrémité basse de l'élément tubulaire inférieur 14. L'extrémité aval du fil 12 peut être par ailleurs pourvue d'un témoin 50 de visualisation, par exemple une perle arrimée sur le fil 12 comme montré à la figure 25.

Le manchon médicamenteux 7 peut incorporer, outre l'agent cytoréducteur, un agent bactériostatique, et éventuellement tous autres agents nécessaires à l'intervention thérapeutique ou opératoire, par exemple un agent opacifiant vis-à-vis des rayons X. Préférentiellement, mais de manière non exclusive, l'agent cytoréducteur est choisi parmi les agents antimitotiques, cytolytiques, les enzymes, les hormones, les anti-enzymes, et les sels métalliques, par exemple des sels d'argent.

Le dispositif thérapeutique représenté aux figures 8 et 9 diffère de celui représenté par référence aux figures 2 à 7, par le fait que les manchons 7 et 17 se confondent avec les éléments tubulaires 3 et 14 respectivement, et plus précisément leur âme 6 définie précédemment.

Le manchon médicamenteux 7 est obtenu par l'incorporation directe au moins en surface de l'agent thérapeutique cytoréducteur dans le matériau, par exemple caoutchouc silicone, de l'âme 6 de l'élément tubulaire 3, dans une zone allongée biologiquement active 5, matérialisée ou non.

L'enveloppe superficielle protectrice 11 est toutefois conservée, de façon à empêcher la libération de l'agent cytoréducteur dans toute partie du dispositif thérapeutique, sans contact avec l'obstacle, dépassant à l'intérieur de la vessie par exemple.

Le dispositif thérapeutique représenté aux figures 10 et 11 diffère de celui représenté aux figures 2 à 7, par le fait que :
- le substrat du manchon médicamenteux 7, dans sa configuration non expansée radialement, présente une surface externe s'inscrivant dans le reste de la surface externe de l'élément tubulaire supérieur 3 ; et dans la position expansée, représentée à la figure 11, la surface externe du manchon médicamenteux 7 émerge de la surface externe du même élément tubulaire 3 ;
- la même conformation, non expansée puis expansée, est retenue pour le manchon extérieur 17 de l'élément tubulaire inférieur 14.

Le dispositif thérapeutique représenté aux figures 12 à 15 diffère de celui représenté par référence aux figures 2 à 7, par le fait que le manchon médicamenteux 7 se limite à une couche superficielle cylindrique du substrat, tandis que le reste de ce dernier constitue un manchon 10 de liaison, expansible, interposé entre l'âme 6 de l'élément tubulaire 3 et le manchon médicamenteux 7 proprement dit. L'élément tubulaire inférieur 14 demeure inchangé.

Le dispositif thérapeutique représenté aux figures 16 et 17 ne diffère de celui représenté par référence aux figures 12 à 15, que par le fait que le manchon médicamenteux 7, mais également le manchon de liaison 10, expansibles, comprennent une pluralité de passages 9 radiaux, de l'extérieur vers l'intérieur du substrat, de manière à permettre et favoriser le passage des liquides ou sécrétions corporelles, contribuant ou augmentant l'expansion de la matière hydrophile. Ces passages radiaux sont également prévus dans l'enveloppe superficielle protectrice 11, aussi bien sur l'élément tubulaire supérieur 3 que sur l'élément tubulaire inférieur 14.

Le dispositif thérapeutique représenté par référence aux figures 18 et 19 ne diffère de celui représenté par référence aux figures 12 à 15, que par le fait qu'il comprend un fourreau 8 d'une mousse synthétique, à la fois compressible et expansible radialement, entre le manchon médicamenteux 7 proprement dit et l'âme 6 de l'élément tubulaire supérieur 3. A l'exclusion du manchon médicamenteux 7, la même disposition est retenue pour le manchon extérieur 17 de l'élément tubulaire inférieur 14.

Un dispositif thérapeutique 1 conforme à la présente invention peut être mis en place dans l'urètre 2, exactement de la même manière que celle décrite dans le document WO-A-94/18907, et en particulier avec les ensembles ou systèmes d'insertion décrits dans ce document. Ces moyens ou dispositifs d'insertion atraumatique sont retirés aussitôt que le dispositif thérapeutique selon la présente l'invention a été inséré dans l'urètre.

En partant de l'urètre représenté schématiquement avant implantation conformément à la figure 20, et d'un dispositif thérapeutique selon l'invention conforme aux figures 2 à 7, le traitement thérapeutique de l'adénome prostatique est obtenu de la manière suivante.

Après insertion atraumatique, le dispositif thérapeutique 1 est positionné par rapport au sphincter 15, par une simple traction du fil 12 d'arrimage. Le contrôle de la bonne position du dispositif thérapeutique peut être obtenu par une échographie par voie endo-rectale. On aboutit à la position représentée à la figure 21.

Au contact des sécrétions naturelles, le dispositif thérapeutique est activé, et l'enveloppe 11 superficielle, recouvrant le manchon médicamenteux 7, se délite au contact direct de l'urètre, de telle sorte que l'urètre puis l'adénome viennent alors au contact direct de la surface extérieure dudit manchon 7. Il en est de même en ce qui concerne le manchon inférieur 17.

Dès lors, les manchons 7 et 17 s'expandent selon toute leur longueur, de façon simultanée, au niveau des éléments tubulaires inférieur 14 et supérieur 3. Cette expansion du manchon 7 augmente la surface de contact entre ce dernier et l'urètre prostatique, en même temps que la pression au niveau de l'adénome en arrière de la muqueuse urétrale. L'expansion du manchon 17 augmente sa section dans l'urètre bulbaire, et améliore la stabilité longitudinale, ou autostatisme du dispositif thérapeutique selon la présente invention. On aboutit ainsi à la configuration du dispositif représentée in situ à la figure 22.

Graduellement, l'érosion de l'urètre 2, puis de l'adénome progresse, selon un front de surface sensiblement cylindrique, centrée sur le manchon médicamenteux 7, tant que le contact mécanique avec la surface extérieure du manchon médicamenteux 7 permet à l'agent cytoréducteur d'exercer son effet chimique sur les tissus environnants. Dès que l'érosion a progressé pour aboutir à une cavité dont la section transversale est sensiblement égale à celle du manchon 7, le contact entre l'agent cytoréducteur et les tissus disparaît, et l'effet thérapeutique cesse.

Dès ce moment, l'urine s'insinue facilement entre le manchon 7 et les tissus, pour imprégner le substrat hydrophile, qui peut être au surplus biodégradable, par exemple sous l'effet d'une modification du pH urinaire. La même dégradation s'observe pour le manchon 17. Dès lors, le substrat hydrophile se délite, pour aboutir à la configuration représentée à la figure 23.

Finalement, le dispositif thérapeutique peut être retiré, dès que l'on souhaite que l'effet thérapeutique cesse, ou lorsque ce dernier est devenu inexistant, en raison de l'absence de contact entre les tissus et le manchon médicamenteux 7. Le retrait du dispositif 1 est possible par une simple traction sur le fil d'arrimage 12, pour aboutir à l'urètre totalement traitée conformément à la figure 24.

Si on utilise un dispositif conforme aux figures 8 et 9, la phase d'expansion est supprimée.

Si on utilise un dispositif conforme aux figures 18 à 19, la phase de résorption des manchons 7 et 17 est supprimée.

L'utilisation d'un dispositif thérapeutique conforme à l'invention apporte une grande sécurité, à la fois passive et active.

S'agissant de la sécurité passive, en ce qui concerne la vessie 20, il n'y a pas de contact direct entre le manchon médicamenteux 7 et la paroi de la vessie. A supposer qu'une partie du manchon 7 émerge dans la vessie et baigne dans l'urine, en l'absence de frottements avec les tissus, l'enveloppe protectrice 11 ne sera pas délitée ou dégradée, ce qui empêche la libération de l'agent cytoréducteur ; et en se déposant sur cette enveloppe, les sels contenus dans l'urine forment une couche protectrice supplémentaire.

S'agissant de la sécurité passive au niveau du sphincter, on notera d'après la description précédente, que celui-ci se trouve à plus de 10 mm du manchon médicamenteux 7. Le segment sous-montanal 211 ne se trouve pas intubé par le manchon médicamenteux 7, et représente une barrière de sécurité pour le sphincter 15. L'urètre bulbaire ne se trouve jamais en contact avec le manchon médicamenteux 7, sauf de façon momentanée au moment de l'insertion du dispositif 1. L'effet cytoréducteur ne peut pas dépasser en profondeur la prostate, puisque comme dit précédemment, il s'agit d'un effet contact ; celui-ci est limité en profondeur par la section du manchon médicamenteux 7, dans sa configuration expansée.

S'agissant de la sécurité active, les moyens de contrôle de la bonne position du dispositif 1 sont :
- pour le médecin, au moment de la mise en place ou d'une visite de contrôle, l'échographie endo-rectale et sus-pubienne, par exemple ; plus tard, le degré de résorption peut être évalué en mesurant le diamètre du manchon médicamenteux, par une radiographie ;
- pour le patient, le fil 12 et son témoin 50 qui s'extériorise au niveau du méat 18, lui permettent de contrôler si le dispositif est en bonne position, à chaque miction ; sa disparition peut lui faire craindre une migration vers le haut, imposant un contrôle médical rapide, et sa migration vers le bas entraîneront une dysurie ou des fuites urinaires imposant également un contrôle médical.

En définitive, le fil 12 a un triple rôle :
- positionnement du dispositif juste après insertion ;
- témoin de la bonne position du dispositif, pendant toute la durée du traitement ;
- et moyen d'extraction du dispositif, à la fin du traitement.

Toute infection pourra être prévenue, par l'incorporation d'un agent bactériostatique dans le manchon médicamenteux 7.

Un dispositif conforme à l'invention apporte par ailleurs un rapport optimal coût-efficacité, pour les raisons suivantes.

Le traitement ne nécessite aucune instrumentation lourde externe, puisque la mise en place peut se faire sans anesthésie générale, avec un simple gel anesthésique de contact dans l'urètre. Et une simple échographie endo_rectale permet le contrôle de la bonne position du dispositif thérapeutique.

La morbidité du dispositif thérapeutique est bien inférieure à celle de la chirurgie, et se réduit à un inconfort périnéal, et à une aggravation transitoire du prostatisme. Mais de toutes façons, la morbidité sera limitée au temps nécessaire à l'action du dispositif thérapeutique, puisque ce dernier est mis en place de manière temporaire.

En termes d'efficacité, avec un dispositif selon l'invention, on crée une cavité de forme appropriée, par exemple cylindrique, à l'intérieur de la prostate, avec un résultat voisin de celui pouvant être obtenu par chirurgie.

Au total, on réussit selon l'invention à combiner l'efficacité maximale (celle de la chirurgie), à la morbidité minimale (celle d'un médicament), pour un coût réduit (celui d'une prothèse).

Selon la présente invention, le dispositif thérapeutique peut avoir une taille unique, puisqu'une longueur de l'élément tubulaire supérieur de l'ordre de 70 mm permet de traiter la majorité des obstacles prostatiques.

Le manchon médicamenteux peut incorporer des composés chimiques non cytostatiques, ni cytolytiques, tels qu'alpha-bloquants, inhibiteurs enzymatiques, enzymes ou hormones, dans le but de réduire l'exposition de l'ensemble de l'organisme à ces agents thérapeutiques, et diminuer leur morbidité tout en conservant une efficacité satisfaisante.

Conformément aux figures 26 et 27, le manchon médicamenteux 7 et l'âme interne 6 sont désaxés l'un par rapport à l'autre, de manière à détruire les cellules proliférantes selon des directions préférentielles.

Un dispositif thérapeutique selon l'invention présente encore les variantes suivantes :
- le manchon souple 16 de liaison entre les éléments tubulaires 3 et 14 peut être ajouré, de manière distribuée sur son pourtour, notamment par des fentes ou fenêtres longitudinales ;
- les éléments tubulaires 3 et 14 forment avec le manchon 16, dans sa conformation d'écoulement, un conduit de section intérieure sensiblement constante selon la direction longitudinale du dispositif ;
- la paroi de chaque élément tubulaire 3 ou 14, et notamment son âme 6, comportent une armature tubulaire, notamment une spirale métallique ou non, par exemple noyée dans le matériau de chaque élément.

## Revendications

1. Dispositif (1) thérapeutique, destiné à un traitement local et sélectif de l'obstruction d'une lumière ou voie naturelle (2) de circulation d'un fluide, disposée dans une partie pleine, notamment un organe solide d'un corps humain ou animal, ladite lumière étant obstruée sous l'effet d'une prolifération cellulaire locale, ledit dispositif comprenant :
- un élément tubulaire (3), non biodégradable, agencé pour être placé dans ladite lumière naturelle, notamment de forme cylindrique, suffisamment souple pour se conformer à ladite lumière naturelle, mais suffisamment rigide pour maintenir un passage artificiel (4) dans ladite lumière ;
- un manchon (7) supporté par ledit élément tubulaire (3), positionné selon la longueur et autour de ce dernier pour venir en regard et au contact direct de l'obstruction, une fois la lumière naturelle intubée par ledit élément tubulaire ;
**caractérisé en ce que** ledit manchon est médicamenteux, car comprenant un agent thérapeutique cytoréducteur, notamment cytotoxique spécifiquement vis-à-vis des cellules de ladite prolifération cellulaire locale, essentiellement par simple contact tissulaire, superficiel et solide, avec lesdites cellules, et est agencé pour délivrer ledit agent thérapeutique au moins dans sa partie superficielle externe.

2. Dispositif thérapeutique selon la revendication 1, caractérisé en ce que le manchon médicamenteux (5) est incorporé dans l'élément tubulaire (3).

3. Dispositif thérapeutique selon la revendication 2, caractérisé en ce que l'élément tubulaire (3) comprend une âme (6) en un matériau biocompatible, notamment relativement lisse et mou, par exemple en un caoutchouc silicone, et dans une zone biologiquement active (5) dudit élément tubulaire correspondant audit manchon médicamenteux (7), le matériau de l'âme (6) incorpore l'agent thérapeutique cytoréducteur, au moins en surface.

4. Dispositif thérapeutique selon la revendication 1, caractérisé en ce que le manchon médicamenteux (7) est distinct de l'élément tubulaire (3), et recouvre ce dernier.

5. Dispositif thérapeutique selon la revendication 4, caractérisé en ce que d'une part l'élément tubulaire (3) comprend une âme (6) interne en un matériau biocompatible, notamment relativement lisse et mou, par exemple en un caoutchouc silicone, et d'autre part le manchon (7) médicamenteux disposé à l'extérieur de l'âme (6), comprend un substrat biologiquement compatible incorporant l'agent thérapeutique cytoréducteur.

6. Dispositif thérapeutique selon la revendication 5, caractérisé en ce que le substrat du manchon (7) médicamenteux est expansible radialement, notamment en sorte que la surface externe dudit manchon peut notamment s'inscrire, en position non expansée, dans le reste de la surface externe de l'élément tubulaire (3), et en position expansée, émerger de la surface externe du même élément tubulaire.

7. Dispositif thérapeutique selon la revendication 6, caractérisé en ce que le substrat du manchon (7) médicamenteux est hydrophile et expansible sous l'effet des fluides biologiques présents ou circulant dans la lumière naturelle obstruée.

8. Dispositif thérapeutique selon la revendication 6, caractérisé en ce qu'il comprend un fourreau (8) d'une mousse synthétique, assurant la liaison entre le manchon médicamenteux (7) et l'âme (6) de l'élément tubulaire (3).

9. Dispositif thérapeutique selon la revendication 5, caractérisé en ce que le manchon médicamenteux (7) comprend une pluralité de passages (9) radiaux, de l'extérieur vers l'intérieur dudit manchon médicamenteux.

10. Dispositif thérapeutique selon la revendication 5, caractérisé en ce qu'il comprend un manchon (10) de liaison, expansible, interposé entre l'âme (6) de l'élément tubulaire (3) et le manchon médicamenteux (7).

11. Dispositif thérapeutique selon la revendication 1, caractérisé en ce qu'il comporte une enveloppe (11) superficielle protectrice du manchon médicamenteux (7), biodégradable par contact tissulaire in situ avec la partie obstruée de la lumière ou voie naturelle.

12. Dispositif thérapeutique selon la revendication 1, caractérisé en ce qu'un fil (12) d'extraction est arrimé à l'extrémité aval (par rapport au sens d'implantation) de l'élément tubulaire (3).

13. Dispositif selon la revendication 1, caractérisé en ce que le manchon médicamenteux (7) comprend un agent bactériostatique, et éventuellement un agent opacifiant vis-à-vis des rayons X.

14. Dispositif thérapeutique selon la revendication 1, caractérisé en ce que l'agent cytoréducteur est choisi parmi les agents antimitotiques, cytolytiques, les enzymes, hormones, anti-enzymes et sels métalliques.

15. Dispositif thérapeutique selon la revendication 5, caractérisé en ce que le manchon médicamenteux (7) et l'âme (6) interne sont désaxés l'un par rapport à l'autre.

16. Dispositif thérapeutique endo-urétral selon l'une quelconque des revendications 1 à 15, destiné au traitement thérapeutique de la partie prostatique (21) de l'urètre de l'homme, caractérisé en ce que le manchon médicamenteux (7) est positionné par rapport à l'élément tubulaire (3) d'une extrémité dite inférieure, située au-dessus de l'extrémité basse (3a) dudit élément tubulaire (3), par exemple à environ 10 mm de ladite extrémité basse, à une extrémité dite supérieure, située en retrait par rapport à l'extrémité haute (3b) dudit élément tubulaire, par exemple à une distance comprise entre 10 et 15 mm de ladite extrémité haute.

17. Dispositif thérapeutique selon la revendication 16, caractérisé en ce que l'extrémité haute (3b) de l'élément tubulaire est borgne, et perforée (13) pour assurer le passage de l'urine à partir de la vessie.

18. Dispositif thérapeutique endo-urétral selon la revendication 16, caractérisé en ce qu'il comprend deux éléments tubulaires (3,14), destinés à être disposés dans l'urètre (2), respectivement de part et d'autre du sphincter (15), et attachés l'un à l'autre par un moyen (16) de liaison, souple et déformable, destiné à être pris dans l'orifice du sphincter, et l'élément tubulaire (3) supérieur supporte le manchon médicamenteux (7) dans la partie (21) prostatique de l'urètre, et l'autre élément tubulaire (14) inférieur ne comporte pas de manchon médicamenteux.

19. Dispositif thérapeutique endo-urétral selon la revendication 18, caractérisé en ce que l'élément inférieur (14) comporte une âme (6) en un matériau biocompatible, mais non biodégradable, notamment relativement lisse et mou, par exemple en caoutchouc silicone.

20. Dispositif thérapeutique selon la revendication 18, caractérisé en ce que l'élément inférieur (14) comporte un manchon extérieur (17), expansible radialement, notamment en sorte que la surface externe dudit manchon extérieur peut s'inscrire , en position non expansée, dans le reste de la surface externe de l'élément inférieur (14), et en position expansée, émerger de la surface externe du même élément inférieur.

## Claims

1. Therapeutic device (1) intended for local and selective treatment of an obstruction in a natural lumen or passage (2) for circulation of a fluid, located in a filled area, in particular in a solid organ of the human or animal body, the said lumen being obstructed by the effect of a local cell proliferation, the said device comprising:
- a non-biodegradable tubular element (3) which is designed to be placed in the said natural lumen, this element being especially of cylindrical shape, and sufficiently flexible to conform to the said natural lumen, but sufficiently rigid to maintain an artificial channel (4) in the said lumen;
- a medicinal sleeve (7) which is supported by the said tubular element (3) and which is positioned along the length of, and around, the latter, so as to come into line with, and into direct contact with, the obstruction once the natural lumen has been intubated with the said tubular element;
characterized in that the said sleeve is medicinal since it comprises a therapeutic agent which is cytoreductive, in particular cytotoxic, specifically vis-à-vis the cells of the said local cell proliferation, essentially through simple superficial and solid tissue contact with the said cells, and designed to deliver the said therapeutic agent at least in its outer surface portion.

2. Therapeutic device according to Claim 1, characterized in that the medicinal sleeve (5 [sic]) is incorporated in the tubular element (3).

3. Therapeutic device according to Claim 2, characterized in that the tubular element (3) comprises a core (6) made of a biocompatible material, in particular a relatively smooth and soft material, for example silicone rubber, and in a biologically active zone (5) of the said tubular element corresponding to the said medicinal sleeve (7) the material of the core (6) incorporates the cytoreductive therapeutic agent, at least on the surface.

4. Therapeutic device according to Claim 1, characterized in that the medicinal sleeve (7) is distinct from the tubular element (3) and covers the latter.

5. Therapeutic device according to Claim 4, characterized in that, on the one hand, the tubular element (3) comprises an internal core (6) made of a biocompatible material, in particular a relatively smooth and soft material, for example silicone rubber, and, on the other hand, the medicinal sleeve (7) arranged outside the core (6) comprises a biologically compatible substrate incorporating the cytoreductive therapeutic agent.

6. Therapeutic device according to Claim 5, characterized in that the substrate of the medicinal sleeve (7) is radially expandable, especially such that the outer surface of the said sleeve can be inscribed in particular, in the non-expanded position, within the remainder of the outer surface of the tubular element (3), and, in the expanded position, can emerge from the outer surface of the same tubular element.

7. Therapeutic device according to Claim 6, characterized in that the substrate of the medicinal sleeve (7) is hydrophilic and expandable under the effect of the biological fluids present or circulating in the obstructed natural lumen.

8. Therapeutic device according to Claim 6, characterized in that it comprises a sheath (8) made of a synthetic foam, ensuring the connection between the medicinal sleeve (7) and the core (6) of the tubular element (3).

9. Therapeutic device according to Claim 5, characterized in that the medicinal sleeve (7) comprises a plurality of radial channels (9) running from the outside toward the inside of the said medicinal sleeve.

10. Therapeutic device according to Claim 5, characterized in that it comprises an expandable connection sleeve (10) interposed between the core (6) of the tubular element (3) and the medicinal sleeve (7).

11. Therapeutic device according to Claim 1, characterized in that it comprises a protective surface envelope (11) for the medicinal sleeve (7), which envelope is biodegradable through tissue contact in situ with the obstructed part of the natural lumen or passage.

12. Therapeutic device according to Claim 1, characterized in that a withdrawal thread (12) is secured to the downstream end (with respect to the direction of implantation) of the tubular element (3).

13. Device according to Claim 1, characterized in that the medicinal sleeve (7) comprises a bacteriostatic agent and, if appropriate, an agent which is opaque vis-à-vis X-rays.

14. Therapeutic device according to Claim 1, characterized in that the cytoreductive agent is chosen from among antimitotic agents, cytolytic agents, enzymes, hormones, antienzymes and metal salts.

15. Therapeutic device according to Claim 5, characterized in that the medicinal sleeve (7) and the internal core (6) are off-centred in relation to one another.

16. Intraurethral therapeutic device according to any one of Claims 1 to 15, intended for the therapeutic treatment of the prostatic portion (21) of the male urethra, characterized in that the medicinal sleeve (7) is positioned, in relation to the tubular element (3), from a so-called lower end, situated above the bottom end (3a) of the said tubular element (3), for example at approximately 10 mm from the said bottom end, to a so-called upper end, situated set back from the top end (3b) of the said tubular element, for example at a distance of between 10 and 15 mm from the said top end.

17. Therapeutic device according to Claim 16, characterized in that the top end (3b) of the tubular element is blind, and is perforated (13) in order to ensure the passage of the urine from the bladder.

18. Intraurethral therapeutic device according to Claim 16, characterized in that it comprises two tubular elements (3, 14) which are intended to be arranged in the urethra (2) on either side, respectively, of the sphincter (15) and are attached to one another by a flexible and deformable connection means (16) which is intended to be held in the orifice of the sphincter, and the upper tubular element (3) supports the medicinal sleeve (7) in the prostatic portion (21) of the urethra, and the other, lower, tubular element (14) does not comprise a medicinal sleeve.

19. Intraurethral therapeutic device according to Claim 18, characterized in that the lower element (14) comprises a core (6) made of a biocompatible but non-biodegradable material, in particular a relatively smooth and soft material, for example silicone rubber.

20. Therapeutic device according to Claim 18, characterized in that the lower element (14) comprises a radially expandable outer sleeve (17), especially such that the outer surface of the said outer sleeve can be inscribed, in the non-expanded position, within the remainder of the outer surface of the lower element (14), and, in the expanded position, can emerge from the outer surface of the same lower element.

## Patentansprüche

1. Therapeutische Vorrichtung (1), bestimmt zur lokalen und selektiven Behandlung einer Obstruktion einer Öffnung oder eines natürlichen Fließweges (2) einer Flüssigkeit, die bzw. der in einem festen Bereich, insbesondere einem soliden Organ des menschlichen oder tierischen Körpers, angeordnet ist, wobei die Öffnung unter dem Einfluß einer lokalen Zellvermehrung obstruiert ist, wobei die Vorrichtung aufweist:
- ein biologisch nicht zersetzbares, röhrenförmiges und vorzugsweise zylindrisches Element (3), das konstruiert ist, um in der natürlichen Öffnung angeordnet zu werden, das biegsam genug ist, um sich der natürlichen Öffnung anzupassen, aber steif genug ist, um in der Öffnung einen künstlichen Durchgang (4) offenzuhalten,
- eine von dem röhrenförmigen Element (3) gehaltene Manschette (7), die über ihre Länge rund um letzteres herum angeordnet ist, um gegenüberliegend zu und in direkten Kontakt mit der Obstruktion zu kommen, sobald die natürliche Öffnung mit dem röhrenförmigen Element intubiert ist,
dadurch gekennzeichnet, daß die Manschette medikamentös ist, indem sie einen therapeutischen, zytoreduzierenden und vorzugsweise speziell in bezug auf die Zellen der lokalen Zellvermehrung zytotoxischen Wirkstoff aufweist, und zwar im wesentlichen bei einfachem Gewebekontakt mit den genannten Zellen, sei er oberflächlich oder solide, und daß die Manschette konstruiert ist, um den Wirkstoff zumindest in ihrem äußeren Oberflächenbereich abzugeben.

2. Therapeutische Vorrichtung nach Anspruch 1, dadurch gekennzeichnet, daß die medikamentöse Manschette (7) in das röhrenförmige Element (3) eingearbeitet ist.

3. Therapeutische Vorrichtung nach Anspruch 2, dadurch gekennzeichnet, daß das röhrenförmige Element (3) einen Kern (6) aus einem biologisch verträglichen, vorzugsweise relativ glatten und weichen Material, wie beispielsweise Siliconkautschuk, aufweist, und zwar in einem der medikamentösen Manschette (7) entsprechenden, biologisch aktiven Bereich (5) des röhrenförmigen Elements, wobei das Material des Kerns (6) den zytoreduzierenden, therapeutischen Wirkstoff zumindest oberflächlich beinhaltet.

4. Therapeutische Vorrichtung nach Anspruch 1, dadurch gekennzeichnet, daß die medikamentöse Manschette (7) von dem röhrenförmigen Element (3) verschieden ist und letzteres bedeckt.

5. Therapeutische Vorrichtung nach Anspruch 4, dadurch gekennzeichnet, daß einerseits das röhrenförmige Element (3) einen inneren Kern (6) aus einem biologisch verträglichen, vorzugsweise relativ glatten und weichen Material, wie beispielsweise Siliconkautschuk, aufweist und daß andererseits die medikamentöse Manschette (7) außen auf dem Kern (6) angeordnet ist, wobei sie ein biologisch verträgliches Substrat aufweist, in das der therapeutische, zytoreduzierende Wirkstoff eingefügt ist.

6. Therapeutische Vorrichtung nach Anspruch 5, dadurch gekennzeichnet, daß das Substrat der medikamentösen Manschette (7) radial ausdehnbar ist, insbesondere derart, daß die Außenfläche der Manschette sich in einer nicht ausgedehnten Position in den Rest der Außenfläche des röhrenförmigen Elements (3) bevorzugt einfügen kann und daß sie in einer ausgedehnten Position über die Außenfläche desselben röhrenförmigen Elements hinausragt.

7. Therapeutische Vorrichtung nach Anspruch 6, dadurch gekennzeichnet, daß das Substrat der medikamentösen Manschette (7) hydrophil und unter dem Einfluß der in der obstruierten natürlichen Öffnung anwesenden oder fließenden biologischen Flüssigkeiten ausdehnbar ist.

8. Therapeutische Vorrichtung nach Anspruch 6, dadurch gekennzeichnet, daß sie eine Lage (8) aus einem synthetischen Schaum aufweist, der die Verbindung zwischen der medikamentösen Manschette (7) und dem Kern (6) des röhrenförmigen Elements (3) gewährleistet.

9. Therapeutische Vorrichtung nach Anspruch 5, dadurch gekennzeichnet, daß die medikamentöse Manschette (7) eine Vielzahl von radialen Durchgängen (9) aufweist, und zwar vom Äußeren zum Inneren der medikamentösen Manschette.

10. Therapeutische Vorrichtung nach Anspruch 5, dadurch gekennzeichnet, daß sie eine ausdehnbare Verbindungsmanschette (10) aufweist, die zwischen dem Kern (6) des röhrenförmigen Elements (3) und der medikamentösen Manschette (7) angeordnet ist.

11. Therapeutische Vorrichtung nach Anspruch 1, dadurch gekennzeichnet, daß sie eine oberflächliche Schutzhülle (11) für die medikamentöse Manschette (7) aufweist, die biologisch durch Gewebekontakt in situ mit dem obstruierten Bereich der Öffnung oder des natürlichen Traktes zersetzbar ist.

12. Therapeutische Vorrichtung nach Anspruch 1, dadurch gekennzeichnet, daß an dem flußabwärts liegenden Ende des röhrenförmigen Elementes (3) (bezogen auf die Richtung des Einsetzens) ein Band (12) zum Herausziehen befestigt ist.

13. Vorrichtung nach Anspruch 1, dadurch gekennzeichnet, daß die medikamentöse Manschette (7) einen bakteriostatischen Wirkstoff und unter Umständen einen gegenüber Röntgenstrahlen undurchsichtigen Wirkstoff aufweist.

14. Therapeutische Vorrichtung nach Anspruch 1, dadurch gekennzeichnet, daß der zytoreduzierende Wirkstoff ausgewählt ist aus antimitotischen Wirkstoffen, zytolytischen Wirkstoffen, Enzymen, Hormonen, Anti-Enzymen und metallischen Salzen.

15. Therapeutische Vorrichtung nach Anspruch 5, dadurch gekennzeichnet, daß die medikamentöse Manschette (7) und der innere Kern (6) in bezug zueinander axial versetzt sind.

16. Endo-urethrale therapeutische Vorrichtung nach irgendeinem der Ansprüche 1 bis 15, bestimmt zur therapeutischen Behandlung des Prostatabereichs (21) des männlichen Harnleiters, dadurch gekennzeichnet, daß die medikamentöse Manschette (7) in bezug auf das röhrenförmige Element (3) angeordnet ist zwischen einem unteren Ende, das oberhalb von dem untersten Ende (3a) des röhrenförmigen Elements (3) gelegen ist, und zwar beispielsweise in ungefähr 10 mm zu diesem untersten Ende, und einem oberen Ende, das in bezug zu dem obersten Ende (3b) des röhrenförmigen Elements zurückgezogen gelegen ist, und zwar beispielsweise in einer Entfernung zwischen 10 und 15 mm zu dem genannten obersten Ende.

17. Therapeutische Vorrichtung nach Anspruch 16, dadurch gekennzeichnet, daß das oberste Ende (3b) des röhrenförmigen Elements blind ist, wobei es mit einem Loch (13) versehen ist, um den Durchgang des Urins ausgehend von der Harnblase zu gewährleisten.

18. Endo-urethrale therapeutische Vorrichtung nach Anspruch 16, dadurch gekennzeichnet, daß sie zwei röhrenförmige Elemente (3, 14) aufweist, die dazu bestimmt sind, in dem Harnleiter (2) jeweils auf einer Seite des Schließmuskels (15) angeordnet zu werden, und die miteinander durch ein biegsames und verformbares Verbindungsmittel (16) verbunden sind, das dazu bestimmt ist, in der Öffnung des Schließmuskels aufgenommen zu werden, wobei das obere röhrenförmige Element (3) die medikamentöse Manschette (7) in dem Prostatabereich (21) des Harnleiters hält und wobei das andere untere röhrenförmige Element (14) keine medikamentöse Manschette aufweist.

19. Endo-urethrale therapeutische Vorrichtung nach Anspruch 18, dadurch gekennzeichnet, daß das untere Element (14) einen Kern (6) aus einem biologisch verträglichen, jedoch nicht biologisch zersetzbaren und vorzugsweise relativ glatten und weichen Material aufweist, beispielsweise aus Siliconkautschuk.

20. Therapeutische Vorrichtung nach Anspruch 18, dadurch gekennzeichnet, daß das untere Element (14) eine radial ausdehnbare Außenmanschette (17) aufweist, insbesondere derart, daß sich die Außenfläche der Außenmanschette in einer nicht ausgedehnten Position in den Rest der Außenfläche des unteren Elements (14) einfügen kann und daß sie in einer ausgedehnten Position über die Außenfläche desselben unteren Elements hinausragt.
